# EUROPEAN PATENT APPLICATION

(11) **EP 2 450 082 A1**
(43) Date of publication of application: **09.05.2012**
(21) Application number: 10306204.8
(22) Date of filing: 03.11.2010
(51) Int. Cl.: A61N 1/39, G06F 19/00, H04M 1/21

(54) **Apparatus comprising a wireless communication device and a defibrillator**

(71) Applicant: Alcatel Lucent, 75007 Paris (FR)
(72) Inventor: Mardoyan, Haik, 91620, Nozay (FR); Tran, Patrice, 91620, Nozay (FR)
(74) Representative: Hervouet, Sylvie

(57) **Abstract**

The invention generally relates to an apparatus (1) comprising a wireless communication device (10), and a defibrillator (2); wherein the apparatus (1) is handheld apparatus, and wherein the apparatus (1) has first electrical connectors (33') to supply electricity having first characteristics for powering said defibrillator (2) and has second electrical connectors (34') to supply electricity having different second characteristics for powering said wireless communication device (10).

## Description

The invention generally relates to wireless communication devices coupled with a defibrillator adapted to deliver electric charges to defibrillate the heart of a victim.

Access to defibrillators in case of emergency is not easy. Defibrillators can usually be found in specific places such as hospitals, airports, railway stations, business centers or schools. This is clearly insufficient. In addition, people are usually not trained to use such defibrillators, thus further reducing their chance to revive a victim using a defibrillator.

Consequently, a real need exists to provide a defibrillator which would be easy to carry and to handle.

It has already been proposed to integrate a defibrillator function within a handheld wireless communication device such as a cellular telephone, thus increasing the number of people who may react rapidly in case of emergency.

However, having a defibrillator embedded in a handheld wireless communication device raises such issues as the way to manage the power supply, the size of the device, the protocol which should be followed for using the defibrillator, etc.

Embodiments of the present invention address those specific issues and provide solutions particularly adapted to a handheld apparatus comprising a wireless communication device and a defibrillator.

A first embodiment of the invention is an apparatus comprising a wireless communication device and a defibrillator, wherein the apparatus is handheld apparatus, and wherein the apparatus has first electrical connectors to supply electricity having first characteristics for powering said defibrillator and has second electrical connectors to supply electricity having different second characteristics for powering said wireless communication device.

In some embodiments, the apparatus further comprises a connected housing, the defibrillator and wireless communication device being contained in the housing, the first and second electrical connectors being located in one or two compartments in the housing.

In some embodiments, the apparatus further comprises a dual core battery comprising a first core having first electrical contacts that connect to said first electrical connectors and a second core having second electrical contacts that connect to said second electrical connectors, the battery being configured to apply a first voltage across the first electrical connectors and to apply a different second voltage across the second electrical connectors.

In other embodiments, the apparatus further comprises:
- a first battery having first electrical contacts that connect to said first electrical connectors and being configured to apply a first voltage across the first electrical connectors and
- a second battery having second electrical contacts that connect to said second electrical connectors and being configured to apply a different second voltage across the second electrical connectors.

The second battery may be rechargeable and the first battery may be not rechargeable. Alternatively, the second battery is rechargeable and the first battery is rechargeable and useable for less than one hour without recharging.

In some embodiments, the apparatus further includes a charger adapted to charge independently and simultaneously either said first core and said second core, or said first battery and said second battery.

According to some other specific features which can be used alone or in combination:
- The defibrillator may comprise at least two electrode pads adapted to be electrically connected to said first electrical connectors through at least two cables.
- The electrode pads and cables are preferably accommodable within a housing of the apparatus, and arranged in such a way that electrode pads are adapted to be extracted from said housing, and cables are adapted to be deployed when said electrode pads are extracted.
- The electrode pads are preferably stored so as to face each other along a thickness direction, and are separated by a layer of conductive gel.
- The electrode pads are gathered in an electrode pack.
- The electrode pads and corresponding cables may be advantageously accommodable within a defibrillator cartridge which is insertable into and removable from said housing. The defibrillator cartridge may be adapted to be enclosed between a front part and a back part of said apparatus. Alternatively, the cartridge may be integrated into a back part of said apparatus.
- The defibrillator may further comprise software adapted to administer and apply a sequence of electrical pulses depending on the type of the victim. For instance, said software may include a software application comprising a dialog asking a user to select the type of victim, e.g., an adult or a child.
- The defibrillator may further comprise inductance selection section for controlling characteristics of pulses depending on the selected type of the victim.

Some embodiments of apparatus in accordance with the present invention are now described, by way of example only, and with reference to the accompanying drawings, in which:
- Figure 1 schematically illustrates a perspective view of an apparatus according to an embodiment of the invention;
- Figures 2 and 3 schematically illustrate two alternative embodiments of a power source particularly adapted to apparatus of figure 1;
- Figure 4 shows an apparatus according to an embodiment, further comprising a charger;
- Figure 5 shows an arrangement of electrode pads in a defibrillator according to an embodiment of the invention;
- Figures 6 to 9 illustrate steps for extracting the electrode pads of figure 5; and
- Figure 10 illustrates an electrical diagram of a control circuit in the defibrillator according to an embodiment of the invention.

In the following description, a wireless communication device is to be understood as any handheld electronic device with communication functionality, such as mobile phones, smart phones, personal digital assistant or PDA, GPS receiver, etc.

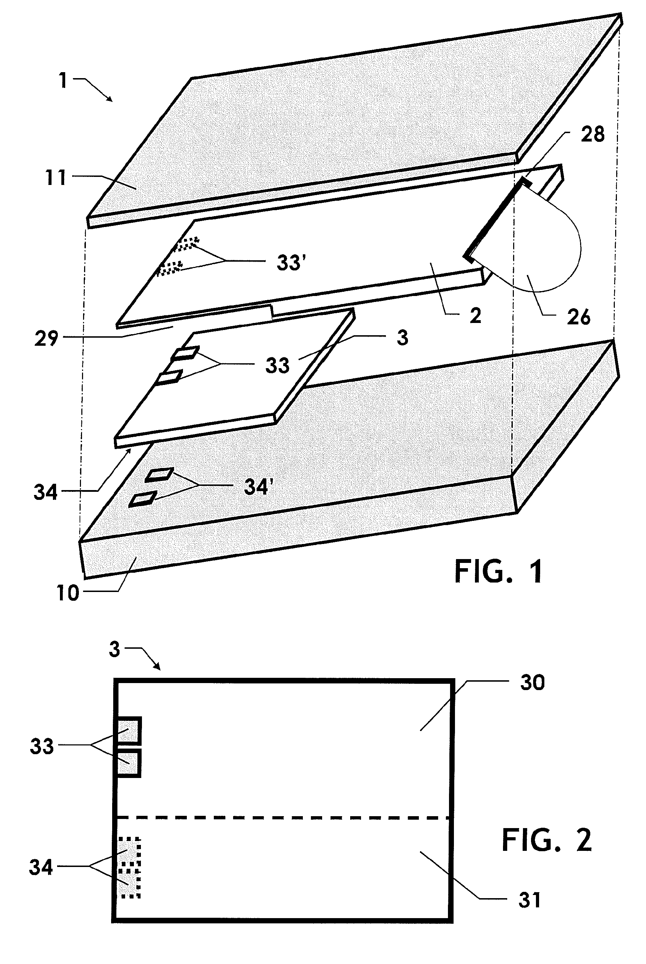

According to an embodiment of the invention schematically illustrated in figure 1, the apparatus 1 comprises a wireless communication device 10, a defibrillator 2 adapted to deliver electric charges to defibrillate the heart of a victim, and power source 3 for supplying power. Such wireless communication device 10, defibrillator 2 and power source 3 may be contained, as shown in figure 1, in a connected housing of apparatus 1, e.g. between a front part 10 and a back part 11 of said housing. Back part 11 is for instance mounted on front part 10 by any known method, such as clipping.

A first aspect of the embodiment concerns the management of the power supply. More precisely, wireless communication device 10 needs electrical power to operate, generally provided by a battery. Defibrillator 2 also needs electrical power. However the electrical characteristics and requirements of power needed for the wireless communication device versus those for the defibrillator are quite different. Indeed, device 10 generally needs low power for a relatively long duration while defibrillator 2 needs higher power for a relatively short duration. Specifically, defibrillator 2 should be able to deliver at least one short pulse, or a precise sequence of short pulses, according to a CPR (Cardiopulmonary Resuscitation) procedure. The voltages needed by both devices are not necessarily the same.

Moreover, normal use of wireless communication device 10 should not completely discharge the power source at the risk of being unable to power a CPR when a heart emergency occurs. Reciprocally, use of the defibrillator function for a CPR should not render device 10 powerless and unusable. This is important to ease users' concern about reliability of the defibrillator function in apparatus 1.

The above problem is solved here by providing energy independence between device 10 and defibrillator 2. To this end, apparatus 1 according to one embodiment comprises first electrical connectors 33' to supply electricity with first characteristics for powering defibrillator 2, and second electrical connectors 34' to supply electricity with second characteristics for powering wireless communication device 10.

First and second electrical connectors 33' and 34' are preferably accommodable in one or two compartments in the housing, like compartment 29 in figure 1.

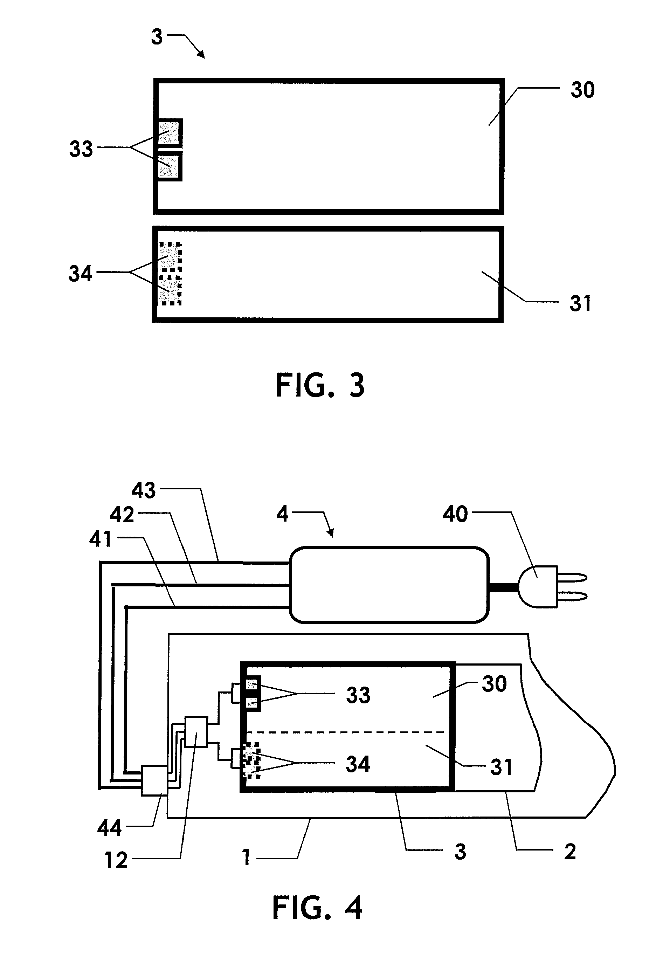

Power source 3 comprises a first supply part 30 (see figures 2 and 3) having first electrical contacts 33 that connect to first electrical connectors 33', and a second supply part 31 having second electrical contacts 34 that connect to second electrical connectors 34'. Power source 3 is configured to apply a first voltage across first electrical connectors 33' and to apply a different second voltage across second electrical connectors 34'.

In other words, different powers are supplied to defibrillator 2 for the defibrillator function and device 10 for other normal communication device functions using the respective, functionally independent supply parts.

First supply part 30 and second supply part 31 may be respectively a first core and a second core of a dual core battery 3, as schematically illustrated in figure 2.

Figure 3 illustrates however an alternative embodiment in which first supply part 30 and second supply part 31 are two distinct batteries, namely a first battery and a second battery. In some cases, only second battery 31 is rechargeable. According to another embodiment, second battery 31 is rechargeable and first battery 30 is rechargeable and useable for less than one hour without recharging.

In all cases, first supply part 30 is adapted to supply to defibrillator 2 the power needed for at least one CPR procedure, while second supply part 31 is dedicated to supply to device 1 the power needed for its normal use. Irrespective of the fact that power source 3 is within the same dual core battery or comprises two distinct batteries, the power source is preferably arranged and adapted to occupy the same space, especially with respect to its thickness, as a usual battery which otherwise would be used for powering the device.

The apparatus according to one embodiment of the invention preferably further includes a charger adapted to charge independently and simultaneously first supply part and second supply part, as will now be described with reference to figure 4. More precisely, figure 4 schematically illustrates the connection between charger 4 and electronic communication device 1, for the purpose of charging both first supply part 30 and second supply part 31.

Charger 4 comprises a first connector 40, e.g., an electric plug, adapted for electrical connection to an electric outlet. Charger 4 is adapted to deliver a first output voltage through a first output cable 41 to charge first supply part 30, and a second output voltage through a second output cable 42 to charge second supply part 31. Cable 43 provides electrical ground reference. Cables 41 through 43 are electrically connected to apparatus 1 through a connector 44, and connected respectively to first and second supply parts 30, 31 through corresponding first electrical contacts 33 and second electrical contacts 34. A control unit 12 between charger 4 and power source 3 controls the charging phase. Said control unit 12 can be part of apparatus 1, as illustrated in figure 4, but could also be part of charger 4. In the first case, said control unit 12 also controls the delivery of power by both first supply part 30 and second supply part 31 through first electrical contacts 33 and second electrical contacts 34 respectively. Control unit 12 is also adapted to recognize whether first supply part 30 is rechargeable or not, and to control the charge of first supply part accordingly.

A second aspect of the embodiment relates to defibrillator 2. Said defibrillator 2 has the following features:
First, electrode pads of the defibrillator provides sufficient surface of contact with the skin of the victim to safely and efficiently apply a sequence of pulses according to a conventional CPR procedure.
Second, the size of the defibrillator is optimized.
Third, as described further below, the operation of defibrillator 2 is simple for a user of apparatus 1.

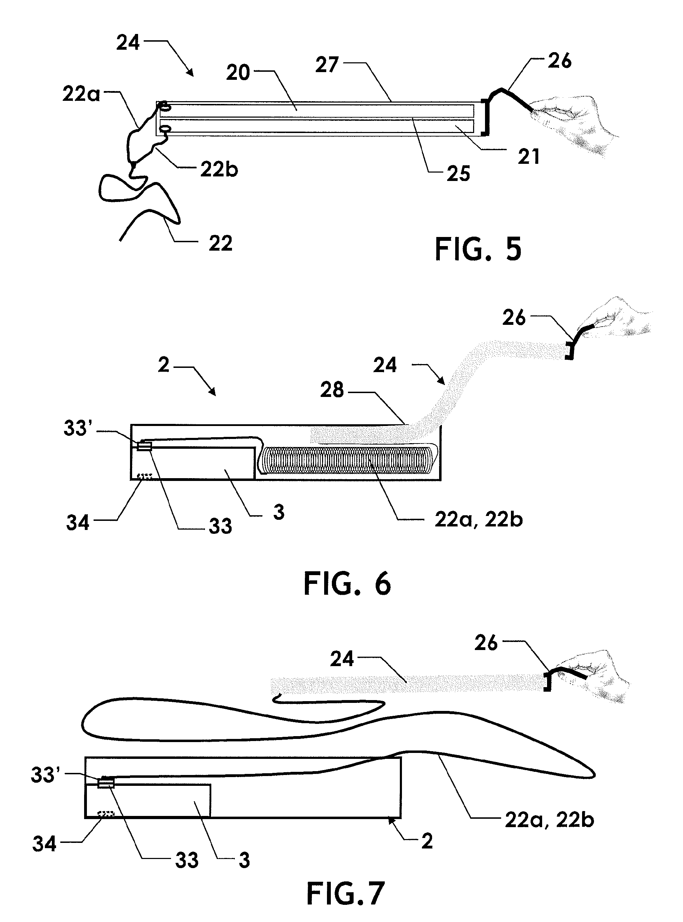

Some embodiments particularly adapted to realize the above features will now be described, with reference to figures 5 to 10:

Defibrillator 2 preferably comprises at least two electrode pads 20, 21 (see figure 5) adapted to be electrically connected to first electrical connectors 33' (see figures 6 and 7) through two corresponding cables 22a, 22b.

Electrode pads 20, 21 and corresponding cables 22a, 22b are located within housing of apparatus 1, and electrode pads are adapted to be extracted from said housing, while corresponding cables 22a, 22b are adapted to be deployed when said electrode pads 20, 21 are extracted. Individual cables 22a, 22b join each other at a certain length to form a Y cable 22, having a common length which allows electrode pads 20, 21 to be sufficiently removed from apparatus 1 and a divided length which allows electrode pads 20, 21 to be sufficiently separated from each other. In one example, said common length is about 1 m and said divided length is at least about 40 cm.

As shown in figure 5, electrode pads 20, 21 are preferably gathered in an electrode pack 24. Said electrode pack 24 preferably comprises a pulling tag 26 for enabling a user to pull electrode pads and cables out of apparatus 1. Electrode pack may also comprise a removable cover 27 protecting at least the outer faces of the electrode pads.

In the embodiment shown, electrode pads are stored so as to face each other along the thickness direction. Specifically, electrode pads 20, 21 are adapted to be stacked back to back (to avoid any electrical short circuit) extending in parallel to back part 11 of apparatus 1 (see figure 1). Owing to this arrangement, it is possible to have electrode pad active surfaces assume the maximum width and length available within apparatus 1, while maintaining a minimal thickness.

Turning back to figure 5, electrode pads 20, 21 are preferably separated by a layer 25 of conductive gel. When electrode pack 24 is extracted, electrode pads 20, 21 are separated from each other, and the conductive gel of layer 25 will advantageously divide into two in order to cover the face of each electrode pad 20, 21. Each electrode pad can then be applied on the skin of a victim, oriented in such a way that the conductive gel is in contact with the skin, thus improving conductivity of the electrical pulses.

According to one embodiment, electrode pads 20, 21 are designed for a single use. To this end, mechanical parts of defibrillator 2, namely electrode pads 20, 21 and corresponding cables 22a, 22b, are housed in a defibrillator cartridge insertable into and removable from housing of apparatus 1, and disposable.

This embodiment is schematically shown in figure 1, where defibrillator 2 is in cartridge form. In this case, cartridge 2 is advantageously provided with a slot 28, through which pulling tag 26 of figure 5 extends.

The single use of a cartridge provides an ergonomic and hygienic solution. Such cartridge may be bought separately in official medical places such as pharmacies. In addition, once electrode pads have been used for a CPR procedure, the need to pack them back into the apparatus is obviated, thus contributing to convenient use of the defibrillator.

Because of the reduced space required by the electrode pads, especially widthwise, cartridge 2 is compact enough to be inserted within apparatus 1, e.g. between the front part 10 and the back part 11 of the apparatus.

Alternatively, the defibrillator cartridge may be integrated into the back part of apparatus 1. In this alternative embodiment, the defibrillator cartridge may contribute to an increase of the apparatus's thickness which preferably is limited to 3 mm or less.

In both cases, removal of the defibrillator cartridge is as easy as removal of a battery.

To further reduce the size of the different parts, namely the defibrillator cartridge and power source 3 (single dual core battery or two distinct batteries), a recess 29 can be advantageously provided in the cartridge housing, said recess 29 forming a compartment adapted to receive, at least in part, said power source 3 (see figure 1). Alternatively, the defibrillator cartridge may also enclose directly all or part of said supply means.

Some of the steps for using electrode pack 24 of figure 5 will now be explained with reference to figures 6 to 9. In these figures, electrode pads 20, 21 and corresponding cable 22a, 22b are located, by way of example, inside a removable cartridge 2. However, the same steps would also apply to the case where electrode pads are stored directly within apparatus 1.

First the user pulls electrode pack 24 out of the defibrillator cartridge using pulling tag 26 until cables 22a, 22b are completely deployed. Figure 6 schematically illustrates the step of pulling electrode pack 24 out of the cartridge 2 while figure 7 schematically represents said electrode pack 24 fully extracted from the cartridge 2, and corresponding cables 22a, 22b deployed. Electrode pack 24 and particularly electrode pads 20, 21 may be composed of flexible materials to facilitate their extraction, as illustrated.

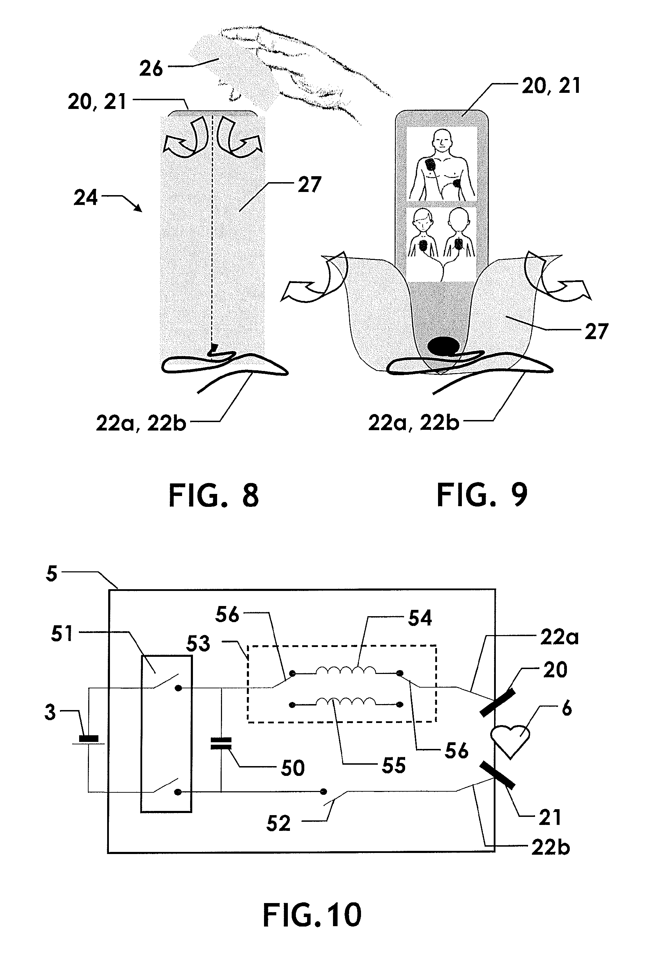

Once electrode pack 24 has been totally extracted and in the position shown in figure 8, the user can remove removable cover 27 as indicated by the arrows. The pulling tag 26 may also be removed. As shown in figure 9, some illustrations giving indications on the best parts of the body electrode pads should be applied on can advantageously be provided directly on electrode pads 20, 21. For instance, a first illustration indicates that, for an adult, the electrode pads should both be placed on the front face of the chest, while a second illustration indicates that, for a child, one electrode pad should be placed on the chest and the other on the back.

A third aspect of the embodiment relates to the software management of the defibrillator function.

In particular, defibrillator 2 further comprises software adapted to administer and apply a sequence of electrical pulses according to a CPR procedure.

Said software includes a software application which may be stored and executed e.g. by a processor available in wireless communication device 10. When started by a user, the software application initiates a dialog with said user typically through a GUI of device 10, to get all the parameters necessary to apply the pulses, guide the user through all the steps to prepare for both the victim and the defibrillator, and then finally apply the pulses.

An aspect of the defibrillator function concerns the ability of the defibrillator to deliver electrical pulses adapted to the type of the victim.

Indeed, an adult and a child should not receive the same electrical treatment during a CPR. The particular power requirements and sequences of electrical pulses used in the respective treatments for an adult and a child are different. For example, the inductance of the electrode pad circuit is also not the same. In known defibrillators, such as the ones used by medical personnel, or the automatic ones that can be found in public places, different sets of electrode pads are provided for an adult and a child, respectively. The choice of the inductance of the electrode pad circuit is thus made by choosing the electrode pads and their associated circuit.

However, this known solution cannot be used in the case where only one set of electrode pads is provided.

To solve this problem, software application according to an embodiment of the invention advantageously comprises a dialog, asking the user to select the type of the victim for which the electrical treatment is intended, e.g., an adult or a child.

Defibrillator 2 also comprises a control circuit used for controlling characteristics of pulses delivered to electrode pads depending on the selected type of victim. Figure 10 schematically illustrates the electrical diagram of a possible control circuit 5 adapted to control a single set of electrode pads, namely electrode pads 20, 21 already disclosed above, depending on the type of victim 6. Control circuit 5 comprises a capacitor 50 adapted to be charged by power source 3 described above. A pair of switches 51, 52 is controlled to charge and discharge the capacitor. Specifically, to charge capacitor 50, switch 51 is switched on while switch 52 is off. Capacitor 50 may discharge and deliver electrical pulses to the electrode pads by switching switch 51 off and switch 52 on simultaneously.

Here, control circuit 5 further comprises inductance selection section 53 for selection between a first inductance 54 and a second inductance 55 adapted for the treatments of a child and an adult respectively. The selection may be done by actuating a toggle switch 56, depending on the type of victim selected by the user, under the control of the processor instructed by the software application.

All the features described above with respect to the different embodiments can be combined together in a single apparatus. However, the arrangements of the electrode pads as described with reference to figures 5 to 9 and/or the software and the control circuit of figure 10 for the selection of the type of victim could also be considered alone.

In addition, other specific features could also be added, especially to the software applications executed by the device, depending on the nature of wireless communication device 1. By way of example, if the device is a cellular phone or any type of device with phone call capabilities, a phone call to an emergency department can be automatically triggered once a user enters into a defibrillator mode. For a cellular phone including GPS localization means, a SMS including the GPS localization of the phone can also be automatically sent.

## Claims

1. An apparatus (1), comprising:
a wireless communication device (10), and
a defibrillator (2);
wherein the apparatus (1) is handheld apparatus, and
wherein the apparatus (1) has first electrical connectors (33') to supply electricity having first characteristics for powering said defibrillator (2) and has second electrical connectors (34') to supply electricity having different second characteristics for powering said wireless communication device (10).

2. The apparatus (1) of claim 1, further comprising a connected housing (10, 11), the defibrillator (2) and wireless communication device (10) being contained in the housing (10, 11), the first and second electrical connectors (33', 34') being located in one or two compartments (29) in the housing.

3. The apparatus (1) of claim 1 or 2, further comprising a dual core battery (3) comprising a first core (30) having first electrical contacts (33) that connect to said first electrical connectors (33') and a second core (31) having second electrical contacts (34) that connect to said second electrical connectors (34'), the battery being configured to apply a first voltage across the first electrical connectors (33') and to apply a different second voltage across the second electrical connectors (34').

4. The apparatus (1) of claim 1 or 2, further comprising
a first battery (30) having first electrical contacts (33) that connect to said first electrical connectors (33') and being configured to apply a first voltage across the first electrical connectors (33') and
a second battery (31) having second electrical contacts (34) that connect to said second electrical connectors (34') and being configured to apply a different second voltage across the second electrical connectors (34').

5. The apparatus (1) of claim 4 wherein the second battery (31) is rechargeable and the first battery (30) is not rechargeable.

6. The apparatus (1) of claim 4 wherein the second battery (31) is rechargeable and the first battery (30) is rechargeable and useable for less than one hour without recharging.

7. The apparatus (1) of claim 3, further including a charger (4) adapted to charge independently and simultaneously said first core (30) and said second (31) of the dual core battery (3).

8. The apparatus (1) according to any one of claims 4 or 6, further including a charger (4) adapted to charge independently and simultaneously said first battery (30) and said second battery (31).

9. The apparatus (1) according to any one of the preceding claims, wherein said defibrillator (2) comprises at least two electrode pads (20, 21) adapted to be electrically connected to said first electrical connectors (33') through at least two cables (22, 22a, 22b).

10. The apparatus (1) according to claim 9, wherein said at least two electrode pads (20, 21) and said cables (22, 22a, 22b) are accommodable within a housing (10, 11) of said apparatus (1), said electrode pads (20, 21) being adapted to be extracted from said housing (10, 11), and said cables (22, 22a, 22b) being adapted to be deployed when said electrode pads (20, 21) are extracted.

11. The apparatus (1) of claim 10, wherein said electrode pads (20, 21) are stored so as to face each other along a thickness direction, and are separated by a layer of conductive gel (25).

12. The apparatus (1) of claim 11, wherein said electrode pads (20, 21) are gathered in an electrode pack (24).

13. The apparatus (1) according to any one of claims 10 to 12, wherein said at least two electrode pads (20, 21) and said cables (22, 22a, 22b) are accommodable within a defibrillator cartridge which is insertable into and removable from said housing (10, 11).

14. The apparatus of claim 13, wherein said defibrillator cartridge is adapted to be enclosed between a front part (10) and a back part (11) of said apparatus (1).

15. The apparatus of claim 13, wherein said defibrillator cartridge is integrated into a back part (11) of said apparatus (1).

16. The apparatus (1) of any one of the preceding claims, wherein said defibrillator (2) further comprises software adapted to administer and apply a sequence of electrical pulses depending on the type of the victim.

17. The apparatus (1) of claim 16, wherein said software includes a software application comprising a dialog asking a user to select a type of victim, e.g., an adult or a child.

18. The apparatus (1) of any one of claims 16 and 17, wherein said defibrillator (2) further comprises inductance selection section (53) for controlling characteristics of pulses depending on the selected type of the victim.
